# FASCICULE DE BREVET EUROPEEN

(11) **EP 1 213 975 B1**
(45) Date de publication et mention de la délivrance du brevet: **03.12.2008**
(21) Numéro de dépôt: 00964326.3
(22) Date de dépôt: 20.09.2000
(51) Int. Cl.: A23L 1/30, A61K 8/97, A61K 31/341, A61Q 5/00, C07C 33/02, C07D 307/36, C07C 29/80

(54) **PROCEDE D'EXTRACTION DES COMPOSES LIPIDES FURANIQUES ET ALCOOLS GRAS POLYHYDROXYLES DE L'AVOCAT**
VERFAHREN ZUR EXTRAKTION VON FURANLIPID-DERIVATEN UND POLYHYDROXYLIERTEN FETTALKOHOLEN DER AVOCADO
METHOD FOR EXTRACTING COMPOUNDS OF FURAN LIPIDS AND POLYHYDROXYLATED FATTY ALCOHOLS OF AVOCADO

(30) Priorité: 22.09.1999 FR 9911846
(43) Date de publication de la demande: 19.06.2002
(73) Titulaire: Laboratoires Expanscience, 92419 Courbevoie Cedex (FR)
(72) Inventeur: BROUTIN, Nicole, F-28800 Alluyes (FR); LEGRAND, Jacques, F-61290 Neuilly sur Eure (FR); PICCIRILLI, Antoine, F-78000 Versailles (FR)
(74) Mandataire: Warcoin, Jacques
(86) Numéro de dépôt international: PCT/FR2000/002601
(87) Numéro de publication internationale: WO 2001/021605

(56) Documents cités:
- EP-A- 0 775 480
- CA-A- 2 213 112
- FR-A- 2 678 614
- A. RANCUREL: "L'avocat: Son huile et son insaponifiable. Utilisation cosmétique" PARFUMS, COSMETIQUES, AROMES., vol. 61, 1985, pages 91-95, XP002144060 SOCIETE D'EXPANSION TECHNIQUE ET ECONOMIQUE S.A. PARIS., FR ISSN: 0337-3029
- M. FARINES ET AL.: "Influence of Avocado Oil Processing on the Nature of Some Unsaponifiable Constituents" JOURNAL OF THE AMERICAN OIL CHEMISTS' SOCIETY., vol. 72, no. 4, 1995, pages 473-476, XP002144061 AMERICAN OIL CHEMISTS' SOCIETY. CHAMPAIGN., US ISSN: 0003-021X cité dans la demande

## Description

La présente invention se rapporte à un nouveau procédé d'extraction des composés lipides furaniques et alcools gras polyhydroxylés de l'avocat.

L'avocat comprend de manière connue des lipides particuliers de type furanique, dont le principal composant est un furane linoléique :

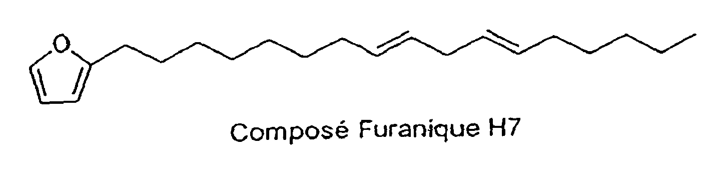

Les dérivés furaniques de l'huile d'avocat ont été décrits notamment dans Farines, M. et al, 1995, J. of Am. Oil Chem. Soc. 72, 473.

Il est aujourd'hui bien établi que la présence de ces composés furaniques dans les feuilles ou le fruit dépend non seulement de la variété (les variétés *Hass et Fuerte* étant les plus riches en composés furaniques) mais aussi du mode d'obtention de l'huile ou d'un autre extrait végétal de l'avocat (extrait hexanique ou éthanolique des feuilles d'avocat).

En effet, on sait que ces lipides furaniques sont des métabolites de composés initialement présents dans le fruit et les feuilles qui, sous l'effet de la chaleur, se déshydratent et se cyclisent en dérivés furaniques.

Par exemple, le furane linoléique est issu de la transformation thermique du précurseur suivant :

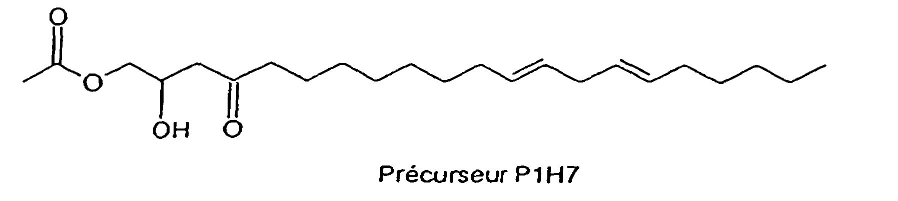

Par ailleurs, certains composés initialement présents dans le fruit et les feuilles de l'avocat peuvent se présenter sous la forme d'alcool gras polyhydroxylés non acétylés, tels que le composé suivant :

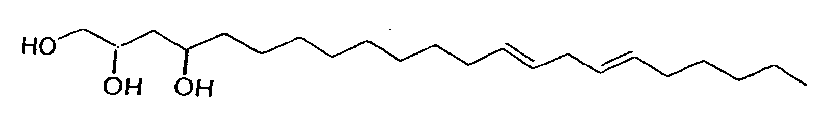

La teneur en alcools gras polyhydroxylés dans le fruit dépend principalement des conditions climatiques, de la qualité des sols, de la saison et de la maturation du fruit à sa cueillette.

De façon générale, les lipides furaniques de l'avocat sont des composés uniques dans le règne végétal et sont surtout recherchés pour leurs propriétés pharmacologiques, cosmétiques, voire nutritionnelles.

Cependant, les techniques connues pour obtenir ces composés spécifiques à partir du fruit ou de l'huile du fruit de l'avocat se résument soit à la chromatographie préparative, soit à des procédés industriels ne permettant d'obtenir ces lipides furaniques qu'en mélange avec les autres composés insaponifiables d'avocat, avec une teneur maximale en lipides furaniques comprise au mieux entre 50 et environ 65% en poids seulement.

En outre, les procédés industriels connus nécessitent une étape préalable de distillation moléculaire de l'huile du fruit pour obtenir les lipides furaniques en des teneurs pourtant encore insatisfaisantes. Cette étape préalable requière la mise en oeuvre de températures élevées telles que des températures supérieures à 180 °C pour des pressions de l'ordre de 10⁻³ mm Hg ce qui, sur le plan industriel, implique une importante consommation d'énergie.

Plus particulièrement, l'obtention de lipides furaniques de plus grande pureté par distillation moléculaire de l'huile du fruit ("brute de pression") est difficile sur le plan industriel compte tenu du caractère très acide de l'huile (indice d'acide d'environ 6 à 10 mgKOH/g) nécessitant une neutralisation préalable par un raffinage au moins partiel de l'huile. De surcroît, un tel raffinage même partiel entraîne une perte conséquente en lipides furaniques et donc une chute du rendement final en ces composés recherchés.

On a maintenant constaté de manière tout à fait surprenante et inattendue que les inconvénients de l'état de la technique décrits ci-dessus peuvent être surmontés par la mise en oeuvre d'un procédé spécifique qui permet d'obtenir une extraction sélective des lipides furaniques de l'avocat avec une teneur de plus de 80% en poids de lipides furaniques, voire proche de 98%.

Par ailleurs, selon une mise en oeuvre particulière du procédé selon l'invention, on obtient avantageusement une extraction sélective non seulement des lipides furaniques mais aussi des alcools gras polyhydroxylés d'avocat.

Qui plus est, le procédé selon l'invention comprend une étape de distillation moléculaire dans laquelle les réglages de températures et de pressions peuvent être nettement inférieures à celles de l'état de la technique précité.

La présente invention a ainsi pour objet un procédé d'extraction sélective des lipides furaniques et des alcools gras polyhydroxylés d'avocat, caractérisé en ce qu'il comprend les étapes consistant à préparer un insaponifiable d'avocat, puis à soumettre l'insaponifiable d'avocat à une étape de distillation moléculaire en utilisant des moyens de température et de pression réglés pour obtenir soit un distillat comprenant principalement des lipides furaniques d'avocat, soit un distillat comprenant principalement des lipides furaniques et des alcools gras polyhydroxylés d'avocat.

« Par lipides furaniques d'avocat », on entend selon l'invention les composants répondant à la formule : 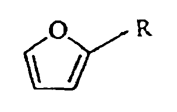 dans laquelle R est une chaîne linéaire hydrocarbonée en C₁₁-C₁₉ de préférence C₁₃-C₁₇ saturée ou comprenant une ou plusieurs insaturations éthyléniques ou acétyléniques.

Par « alcool gras polyhydroxylé d'avocat » on entend selon l'invention un polyol sous forme d'une chaîne principale linéaire hydrocarbonée en C₁₇ - C₂₁ saturée ou comprenant une ou plusieurs insaturations éthyléniques ou acétyléniques, et comprenant au moins deux groupes hydroxyles, les groupes hydroxyles étant majoritairement situés sur une partie de la chaîne principale, de préférence vers l'une des deux extrémités de la chaîne principale, l'autre partie de cette chaîne principale constituant ainsi la chaîne « grasse » (partie hydrophobe) du polyol.

L'insaponifiable est la fraction d'un corps gras qui, après action prolongée d'une base alcaline, reste insoluble dans l'eau et peut être extraite par un solvant organique. Cinq grands groupes de substances sont présents dans la plupart des insaponifiables d'huiles végétales : hydrocarbures saturés ou insaturés, alcools aliphatiques ou terpéniques, stérols, tocophérols, les pigments caroténoïdes et xanthophiles.

La comparaison des teneurs en insaponifiables de différentes huiles végétales : soja, coton, noix de coco, olive et avocat montre un taux très important d'insaponifiable de l'huile d'avocat obtenue par extraction suivant divers procédés connus. Typiquement, les teneurs obtenues s'échelonnent de 2 à 7% d'insaponifiable dans l'huile d'avocat contre 0,5% dans l'huile de coco, 1% dans l'huile de soja, 1% dans l'huile d'olive. L'insaponifiable d'avocat peut être préparé par extraction à partir de l'huile d'avocat.

Les procédés connus d'obtention de l'huile d'avocat sont principalement les suivants :
- soit on presse la pulpe fraîche en présence d'un tiers corps fibreux absorbant l'eau tel que la parche de café dans une presse à cage, puis on sépare l'émulsion d'huile et d'eau obtenue par décantation et/ou centrifugation;
- soit on broie la pulpe fraîche et on la met en contact avec un solvant organique adapté (par exemple un mélange méthanol-chloroforme) puis on récupère l'huile par évaporation du solvant.

L'huile d'avocat ainsi obtenue est ensuite soumis à une extraction de l'insaponifiable de manière connue.

Plusieurs procédés ont été décrits pour extraire l'insaponifiable d'une huile végétale. Tous retiennent de préférence la saponification par la potasse ou la soude en milieu alcoolique, de préférence éthanolique, suivie d'une ou plusieurs extraction(s) par un solvant organique approprié, par exemple l'éther de pétrole, l'éther éthylique ou tout autre solvant approprié non miscible avec la solution hydroalcoolique.

La solution d'extraction obtenue est de préférence ensuite centrifugée, filtrée puis lavée à l'eau pour éliminer les traces résiduelles d'alcalinité. Enfin, le solvant d'extraction est évaporé soigneusement pour récupérer l'insaponifiable. On peut également bien entendu prévoir des opérations supplémentaires connues de l'homme du métier, telle qu'une étape de désodorisation.

De préférence, l'insaponifiable d'avocat est préparé à partir du fruit préalablement traité thermiquement, avant l'extraction de l'huile et la saponification, comme décrit en particulier dans la demande de brevet FR-91 08301.

Ce traitement thermique consiste en un séchage contrôlé du fruit, frais de préférence, pendant au moins quatre heures, avantageusement au moins 10 heures, de préférence entre environ 24 et environ 48 heures, à une température de préférence d'au moins environ 80 °C et de préférence comprise entre environ 80 et environ 120 °C.

On comprend bien entendu que la température et le temps de séchage sont deux paramètres liés l'un à l'autre quant au résultat escompté du traitement thermique qui est de promouvoir la cyclisation des précurseurs des lipides furaniques.

Engin, avant sa saponification, l'huile peut être préalablement enrichie en insaponifiable en séparant une majorité des constituants de l'insaponifiable que l'on récupère dans un concentrat. Différentes méthodes peuvent être utilisées :
cristallisation par le froid, extraction liquide-liquide, distillation moléculaire. La concentration préalable de l'huile en insaponifiable permet de diminuer Ja consommation d'huile pendant la saponification. La distillation moléculaire est particulièrement préférée, étant réalisée de préférence à une température comprise entre environ 180 et environ 230 °C en maintenant une pression comprise entre 10⁻³ et 10⁻² mmHg et de préférence de l'ordre de 10⁻³ mmHg. La concentration en insaponifiable du distillat peut ateindre 60%.

D'une façon générale, la composition moyenne d'un insaponifiable d'avocat obtenu principalement par séchage contrôlé du fruit, extraction de l'huile par pression à froid, distillation moléculaire préalable de l'huile avant saponification par potasse éthanolique, extraction de l'insaponifiable dans une colonne à contre-courant par un solvant organique, filtration, lavage, désolvatation et désodorisation, est la suivante (en pourcentages en poids par rapport au poids total de l'insaponifiable) :
- alcools gras polyhydroxylés 5-25 %
- lipides furaniques 50-70 %
- stérols 2-4 %
- squalène 0,5-5 %
- autres 5-20%(1)
(1) acides gras libres, hydrocarbures, tocophérols, cétones grasses et pigments lourds

Selon l'invention, l'insaponifiable d'avocat obtenu comme décrit ci-dessus est ensuite soumis à une étape de distillation moléculaire.

Selon l'invention, cette étape de distillation moléculaire, dont la combinaison avec l'étape préalable de préparation de l'insaponifiable constitue une caractéristique essentielle du présent procédé, est réalisée avec des moyens de température réglés pour une température comprise entre 100 et 160°C et des moyens de pression réglés pour une pression comprise entre 10⁻³ et 5.10⁻² mmHg.

En particulier, les moyens de température sont réglés pour une température comprise entre 100 et 140 °C et les moyens de pression sont réglés pour une pression comprise entre 10⁻³ et 5.10⁻² mmHg, pour obtenir un distillat comprenant principalement des lipides furaniques d'avocat.

Par ailleurs, selon une variante avantageuse du présent procédé, les moyens de température sont réglés pour une température comprise entre 130 et 160 °C et les moyens de pression sont réglés pour une pression comprise entre 10⁻³ et 5.10⁻² mmHg, pour obtenir un distillat comprenant principalement des lipides furaniques d'avocat et des alcools gras polyhydroxylés d'avocat.

Cette étape de distillation moléculaire de l'insaponifiable, ainsi que toutes autres distillations moléculaires pouvant être mise en oeuvre dans le procédé de l'invention, comme décrit ci-dessus, sont de préférence réalisées en utilisant un dispositif choisi parmi les distillateurs moléculaires de type centrifuge et les dispositifs moléculaires de type à film raclé.

Les distillateurs moléculaires de type centrifuge sont connus de l'homme du métier. Par exemple, la demande EP- 0 493 144 décrit un distillateur moléculaire de ce type. D'une manière générale, le produit à distiller est étalée en couche mince sur la surface chauffée (surface chaude) d'un rotor conique tournant à grande vitesse. L'enceinte de distillation est placée sous vide. Dans ces conditions, il y a évaporation et non pas ébullition, depuis la surface chaude, des constituants de l'insaponifiable, l'avantage étant que l'huile et l'insaponifiable (ces produits étant réputés fragiles) ne sont pas dégradés au cours de l'évaporation.

Les distillateurs moléculaires de type à film raclé, également connus de l'homme du métier, comprennent une chambre de distillation dotée d'un racleur tournant, permettant l'étalement en continu sur la surface d'évaporation (surface chaude) du produit à distiller. Les vapeurs de produit sont condensées par le biais d'un doigt réfrigéré, placé au centre de la chambre de distillation. Les systèmes périphériques d'alimentation et de vide sont très proches de ceux d'un distillateur centrifuge (pompes d'alimentation, pompes à vide à palette et à diffusion d'huile,etc.). La récupération des résidus et des distillats dans des ballons en verre, se fait par écoulement gravitationnel.

Les distillats comprenant principalement des lipides furaniques d'avocat et les distillats comprenant principalement des lipides furaniques et des alcools gras polyhydroxylés d'avocat, tels qu'obtenus par le procédé décrit ci-dessus, peuvent être utilisés en tant que principes actifs dans une composition pharmaceutique avec au moins un excipient pharmaceutiquement acceptable

Plus particulièrement, les distillats comprenant principalement des lipides furaniques d'avocat et les distillats comprenant principalement des lipides furaniques et des alcools gras polyhydroxylés d'avocat, tels qu'obtenus par le procédé décrit ci-dessus, peuvent être utilisés pour la fabrication d'un médicament destiné au traitement des affections des articulations, plus particulièrement au traitement de l'arthrose et au traitement des arthrites (c'est à dire l'arthrite rhumatoïde, l'arthrite psoriasique, l'arthrite de Lyme et/ou toute autre type d'arthrite).

Le médicament ainsi préparé peut être destiné au traitement des affections parodontales, et en particulier au traitement de la périodontite.

Ce médicament peut par ailleurs être destiné au traitement de l'ostéoporose.

En outre, ce médicament peut être destiné à moduler la différenciation des cellules nerveuses induites par le NGF. Par "moduler", on entend selon l'invention l'action d'augmenter ou de diminuer la différenciation des cellules nerveuses induites par le NGF.

Enfin, ce médicament peut être destiné à la réparation tissulaire, et en particulier à la réparation tissulaire cutanée, notamment dans le cadre d'une application dermatologique.

Les distillats comprenant principalement des lipides furaniques d'avocat et les distillats comprenant principalement des lipides furaniques et des alcools gras polyhydroxylés d'avocat, tels qu'obtenus par le procédé décrit ci-dessus, peuvent être utilisés en tant que principes cosmétiquement actifs dans une composition cosmétique, notamment dermo-cosmétique, avec au moins un excipient cosmétiquement acceptable.

Une composition cosmétique comprenant au moins un composé choisi dans le groupe constitué par les distillats comprenant principalement des lipides furaniques d'avocat et les distillats comprenant principalement des lipides furaniques et des alcools gras polyhydroxylés d'avocat, tels qu'obtenus par le procédé décrit ci-dessus, et au moins un véhicule cosmétiquement acceptable tel que les véhicules généralement utilisés dans le domaine des produits cosmétiques, peut être utilisée dans une méthode de traitement de la peau, des muqueuses voisines et/ou des phanères.

De préférence, il s'agit d'une méthode de traitement cosmétique des cicatrices de la peau, du vieillissement intrinsèque de la peau (c'est-à-dire du vieillissement de la peau ne résultant pas majoritairement d'une action extérieure à la peau) et d'une méthode de traitement cosmétique de la peau ayant été soumise à un rayonnement actinique, notamment à un rayonnement ultra-violet.

De préférence, l'insaponifiable d'huile végétale est présent dans la composition cosmétique selon une proportion comprise entre environ 0,1 et environ 10 % en poids, par rapport au poids total de la composition cosmétique.

Enfin, les distillats comprenant principalement des lipides furaniques d'avocat et les distillats comprenant principalement des lipides furaniques et des alcools gras polyhydroxylés d'avocat, tels qu'obtenus par le procédé décrit ci-dessus, peuvent être utilisés dans un additif alimentaire.

Enfin, au moins un composé choisi dans le groupe constitué par les distillats comprenant principalement des lipides furaniques d'avocat et les distillats comprenant principalement des lipides furaniques et des alcools gras polyhydroxylés d'avocat, tels qu'obtenus par le procédé d'extraction sélective décrit ci-dessus, peut être utilisé en tant qu'additif dans un aliment pour l'être humain et/ou l'animal, selon une proportion dudit composé comprise entre environ 0,1 et environ 20 % en poids, par rapport au poids total de l'aliment.

Les exemples suivants sont destinés à illustrer la présente invention et ne doivent en aucun cas être interprétés comme pouvant en limiter la portée.

A moins qu'il n'en soit précisé autrement, les pourcentages indiqués dans les exemples suivants sont des pourcentages en poids.

### Exemple 1 : préparation d'un insaponifiable d'avocat

100 g d'avocat coupé en lamelles de 5 mm d'épaisseur environ sont soumis aux opérations suivantes :

### 1.1) Traitement thermique du fruit

On place dans une étuve régulée à 80 °C les fruits découpés pendant 24 heures, puis on les broie.

### 1.2) Obtention de l'huile

La poudre obtenue à l'étape précédente est extraite à l'hexane ou par pression à froid dans une presse à vis de type « KOMET ». Le tourteau est éliminé et la solution hexanique est évaporée sous pression réduite. L'huile récupérée est filtrée sur «Büchner » puis stockée sous azote. On obtient ainsi 20 g d'huile d'avocat.

### 1.3) Concentration

On utilise un distillateur à film raclé tel que décrit ci-dessus, commercialisé par la société Leybold sous la dénomination "KDL 4". Il s'agit d'un appareil en verre, équipé d'une chambre de distillation dotée d'un racleur tournant, permettant J'étalement en continu sur la surface d'évaporation (surface chaude) du produit à traiter. Les vapeurs de produit sont condensées par le biais d'un doigt réfrigéré, placé au centre de la chambre de distillation. Les systèmes périphériques d'alimentation et de vide sont très proches de ceux d'un distillateur centrifuge (pompes d'alimentation, pompes à vide à palette et à diffusion d'huile, ...). La récupération des résidus et des distillats dans des ballons en verre, se fait par écoulement gravitationnel.

La température de distillation est d'environ 230 °C avec une pression de l'ordre de 10⁻³ mmHg. La vitesse de rotation de l'arbre est de 200 t/min et le débit d'alimentation est de 7 ml/mn.

### 1.4) Saponification

50 g de concentrat d'huile obtenu comme à l'étape précédente sont mélangés à 25 ml de potasse 12N, 100 ml d'éthanol et mis au reflux pendant 4 heures. On ajoute 175 ml d'eau à la phase hydroalcoolique, puis on ajoute 175 ml de dichloroéthane et on agite, puis on laisse décanter. On récupère ensuite la phase organique. Cette opération est répétée 5 à 6 fois. Les phases organiques sont réunies, lavées à l'eau et le solvant évaporé. On obtient ainsi 20 g d'insaponifiable. On peut bien entendu, pour une préparation à l'échelle industrielle, remplacer les étapes d'extraction en ampoule par une extraction en continu dans un apareil d'extraction liquide-liquide en continu tel que colonne puisée, mélangeur décanteur ou équivalents.

### Exemple 2 : distillation moléculaire d'un insaponifiable riche en alcools gras polyhydroxylés

On prépare un insaponifiable comme décrit ci-dessus. L'analyse de cet insaponifiable montre qu'il est riche en alcools gras polyhydroxylés (de l'ordre de 25%). Sa composition est la suivante :
- alcools gras polyhydroxylés 24,3 %
- lipides furaniques 55,5 %
- stérols 3,1%
- squalène 1,4 %
- autres 15,7 % (1)
(1) acides gras libres, hydrocarbures, tocophérols, cétones grasses et pigments lourds

### 2.1) Distillation en laboratoire

On soumet cet insaponifiable à une distillation moléculaire à l'aide du même appareil à film raclé "KDL4" de la société Leybold décrit ci-dessus. Les conditions de distillation sont par contre les suivantes :
- température surface chaude: 108°C
- pression : 10⁻³ mm Hg
- vitesse de rotation de l'arbre: 240 t/min.
- débit d'alimentation d'insaponifiable d'avocat: 400 ml/h

Rendement en distillat: 48,6 %
Composition du distillat:
   - alcools gras Polyhydroxylés : n.m.
   - lipides furaniques 99,1 %
   - stérols n.m.
   - squalène n.m.
   - autres 0,9 % (1)
(1) acides gras libres, hydrocarbures et cétones grasses
("n.m." : non mesurable, c'est à dire une teneur inférieure à 0,05 %)

Il s'agit donc d'un distillat très riche en lipides furaniques dans la mesure où la teneur de ces dernier excède 99 %

### 2.2) Distillation moléculaire sur pilote

Le même insaponifiable d'avocat est distillé dans un distillateur moléculaire pilote (15-25 kg/h) de type centrifuge, fonctionnant en continu, sous un vide compris entre 0,01 et 0,05 mm de mercure, et à une température comprise dans l'intervalle de 100 à 150°C.

Les résultats obtenus à différentes températures et pressions sont rassemblés dans les tableaux 1 et 2 suivants.

**Tableau 1 : Extraction des lipides furaniques par distillation moléculaire d'un insaponifiable d'avocat riche en alcools gras polyhydroxylés**

| Echantillon | Température (T °C) | Pression (mm Hg) | Débit d'alimentation (kg/h) | Taux de distillation (%) | Teneur en lipides furaniques dans le distillat (%) |
|---|---|---|---|---|---|
| 1 | 129 | 0,020 | 13,3 | 60,0 | 93,2 |
| 2 | 123 | 0,025 | 13,5 | 53,6 | 96,2 |
| 3 | 112 | 0,050 | 12,9 | 47,0 | 97,7 |

Le taux de distillation est définie de la manière suivante : il s'agit du rapport massique ramené à 100 % de la masse du distillat à la somme (masse du distillat + masse du résidu).

Les résultats rassemblés dans le tableau 1 démontrent que la distillation moléculaire d'un insaponifiable d'avocat permet de préparer de façon simple et avec un rendement appréciable, une fraction très riche en lipides furaniques de l'avocat (teneur supérieure à 97%).

De façon identique, les résultats des essais rassemblés dans le tableau 2 montrent qu'il est aussi possible d'obtenir des fractions sélectivement enrichies en avocadofuranes et en alcools gras polyhydroxylés.

**Tableau 2 : Extraction des lipides furaniques et des alcools gras polyhydroxylés par distillation moléculaire d'un insaponifiable d'avocat riche en alcools gras polyhydroxylés**

| Echantillon | T (°C) | Pression (mm de Hg) | Débit d'alimentation (kg/h) | Taux de distillation (%) | Teneur en lipides furaniques dans le distillat (%) | Teneur en alcools gras polyhydroxylés dans le distillat (%) |
|---|---|---|---|---|---|---|
| 4 | 150 | 0,015 | 12,8 | 79,8 | 73,5 | 22,0 |
| 5 | 137 | 0,025 | 12,6 | 85,5 | 66,9 | 25.5 |

En conclusion, l'ensemble de ces essais montre qu'il est possible par distillation moléculaire d'un insaponifiable d'avocat, riche en alcools gras polyhydroxylés, de préparer des distillats de très grande pureté, comprenant sélectivement une forte teneur en lipides furaniques (pureté supérieure à 97 %) ou une forte teneurs en lipides furaniques avec des alcools gras polyhydroxylés (somme des teneurs en lipides furaniques et en alcools gras polyhydroxylés supérieure à 95 %).

### Exemple 3 : distillation moléculaire d'un-insaponiriable pauvre en alcools gras polyhydroxylés

On prépare un insaponifiable comme décrit ci-dessus. L'analyse de cet insaponifiable montre qu'il est pauvre en alcools gras polyhydroxylés (de l'ordre de 4%). Sa composition est la suivante :
- alcools gras polyhydroxylés 3,7 %
- lipides furaniques 68,7 %
- stérols 6,6 %
- squalène 1,4 %
- autres 19,6% (1)
(1) acides gras libres, hydrocarbures, tocophérols, cétones grasses et pigments lourds

### 3.1) Distillation en laboratoire

On soumet cet insaponifiable à une distillation moléculaire à l'aide du même appareil à film raclé "KDL4" de la société Leybold que celui déjà décrit ci-dessus.
Les conditions de distillation sont les suivantes :
- température surface chaude: 108 °C
- pression : 10⁻³ mm Hg
- vitesse de rotation de l'arbre : 240 t/min
- Débit d'alimentation insaponifiable d'avocat: 400 ml/h

Rendement en distillat : 50,8 %
Composition du distillat :
   - alcools gras polyhydroxylés 0,2 %
   - lipides furaniques 94,8 %
   - stérols 0,1 %
   - squalène 0,2 %
   - autres 4,7 %

Il s'agit donc d'un distillat très riche en lipides furaniques dans la mesure ou la teneur de ces dernier excède 94%.

### 2.2) Distillation moléculaire sur pilote

Le même insaponifiable d'avocat est distillé dans un distillateur moléculaire pilote (15-25 kg/h) de type centrifuge, fonctionnant en continu, sous un vide compris entre 0,01 et 0,05 mm de mercure, et à une température comprise dans l'intervalle 100 et 140°C. Les résultats obtenus à différentes températures et pression sont rassemblés dans le tableau 3 suivant.

**Tableau 3 : Extraction des lipides furaniques par distillation moléculaire d'un insaponifiable d'avocat pauvre en alcools gras polyhydroxylés**

| Echantillon | T (°C) | Pression (mm de Hg) | Débit d'alimentation (kg/h) | Taux de distillation (%) | Teneur en composés furaniques dans le distillat (%) |
|---|---|---|---|---|---|
| 1 | 115 | 0,015 | 20,4 | 47,8 | 94,3 |
| 2 | 126 | 0,015 | 18,6 | 65,8 | 94,2 |
| 3 | 137 | 0,415 | 18,5 | 71,3 | 93,8 |

Les résultats rassemblés dans le tableau 3 démontrent que la distillation moléculaire d'un insaponifiable d'avocat permet de préparer de façon simple et avec un rendement appréciable, un distillat très riche en lipides furaniques de l'avocat (teneur supérieure à 94 %).

## Revendications

1. Procédé d'extraction sélective des lipides furaniques et des alcools gras polyhydroxylés d'avocat, **caractérisé en ce qu'**il comprend les étapes consistant à préparer un insaponifiable d'avocat, puis à soumettre l'insaponifiable d'avocat à une étape de distillation moléculaire en utilisant des moyens de température et de pression réglés pour obtenir soit un distillat comprenant principalement des lipides furaniques d'avocat, soit un distillat comprenant principalement des lipides furaniques et des alcools gras polyhydroxylés d'avocat, la température de distillation moléculaire étant comprise entre 100 et 160 °C et la pression de distillation moléculaire étant comprise entre 10⁻³ et 5.10⁻² mmHg.

2. Procédé selon la revendication 1, **caractérisé en ce que**, pour l'étape de distillation moléculaire suivant celle de préparation de l'insaponifiable, les moyens de température sont réglés pour une température comprise entre 100 et 140 °C et les moyens de pression sont réglés pour une pression comprise entre 10⁻³ et 5.10⁻² mmHg, pour obtenir un distillat comprenant principalement des lipides furaniques d'avocat.

3. Procédé selon selon la revendication 1, **caractérisé en ce que**, pour l'étape de distillation moléculaire suivant celle de préparation de l'insaponifiable, les moyens de température sont réglés pour une température comprise entre 130 et 160 °C et les moyens de pression sont réglés pour une pression comprise entre 10⁻³ et 5.10⁻² mmHg, pour obtenir un distillat comprenant principalement des lipides furaniques d'avocat et des alcools gras polyhydroxylés d'avocat.

4. Procédé selon l'une quelconque des précédentes revendications, **caractérisé en ce que** l'insaponifiable d'avocat est préparé à partir du fruit préalablement traité thermiquement.

5. Procédé selon la revendication 4, **caractérisé en ce que** le fruit est préalablement traité thermiquement par séchage contrôlé à une température d'au moins environ 80 °C pendant un temps d'au moins environ quatre heures.

6. Procédé selon l'une quelconque des revendications précédentes, **caractérisé en ce que** l'insaponifiable d'avocat est préparé à partir de l'huile du fruit préalablement enrichie en insaponifiable par distillation moléculaire.

7. Procédé selon la revendication 6, **caractérisée en ce que** la distillation moléculaire de l'huile du fruit est réalisée à une température comprise entre environ 180 et environ 230 °C et à une pression comprise entre environ 10⁻³ et environ 10⁻² mmHg.

8. Procédé selon l'une quelconque des revendications précédentes, **caractérisé en ce que** la distillation moléculaire est réalisée en utilisant un dispositif choisi parmi les distillateurs moléculaires de type centrifuge et les dispositifs moléculaires de type à film raclé.

## Claims

1. Process for selectively extracting the furan lipids and polyhydroxylated fatty alcohols of avocado, **characterized in that** it comprises the steps consisting in preparing an unsaponifiable material from avocado, and then in subjecting the unsaponifiable material from avocado to a step of molecular distillation using temperature and pressure means that are adjusted so as to obtain either a distillate mainly comprising furan lipids of avocado, or a distillate mainly comprising furan lipids and polyhydroxylated fatty alcohols of avocado, the molecular distillation temperature being between 100 and 160°C and the molecular distillation pressure being between 10⁻³ and 5 x 10⁻² mmHg.

2. Process according to Claim 1, **characterized in that**, for the molecular distillation step following that of preparation of the unsaponifiable material, the temperature means are adjusted for a temperature of between 100 and 140°C and the pressure means are adjusted for a pressure of between 10⁻³ and 5 x 10⁻² mmHg, so as to obtain a distillate mainly comprising furan lipids of avocado.

3. Process according to Claim 1, **characterized in that**, for the molecular distillation step following that of preparation of the unsaponifiable material, the temperature means are adjusted for a temperature of between 130 and 160°C and the pressure means are adjusted for a pressure of between 10⁻³ and 5 x 10⁻² mmHg, so as to obtain a distillate mainly comprising furan lipids of avocado and polyhydroxylated fatty alcohols of avocado.

4. Process according to any one of the preceding claims, **characterized in that** the unsaponifiable material from avocado is prepared from the fruit that has been heat-treated beforehand.

5. Process according to Claim 4, **characterized in that** the fruit is heat-treated beforehand by controlled drying at a temperature of at least about 80°C for a period of at least about four hours.

6. Process according to any one of the preceding claims, **characterized in that** the unsaponifiable material from avocado is prepared from the oil of the fruit that is enriched beforehand in unsaponifiable material by molecular distillation.

7. Process according to Claim 6, **characterized in that** the molecular distillation of the oil of the fruit is performed at a temperature of between about 180 and about 230°C and at a pressure of between about 10⁻³ and about 10⁻² mmHg

8. Process according to any one of the preceding claims, **characterized in that** the molecular distillation is performed using a device chosen from molecular distillation devices of centrifugal type and molecular devices of scraped-film type.

## Patentansprüche

1. Verfahren zur selektiven Extraktion der furanischen Lipide und der polyhydroxylierten Fettalkohole von Avocado, **dadurch gekennzeichnet, dass** es die Schritte umfasst, die daraus bestehen, einen unverseifbaren Anteil von Avocado herzustellen, dann den unverseifbaren Anteil von Avocado einem Molekulardestillationsschritt zu unterwerfen unter Verwendung von Temperatur- und Druckmitteln, welche so eingestellt werden, dass man entweder ein Destillat, welches hauptsächlich furanische Lipide von Avocado umfasst, oder ein Destillat, welches hauptsächlich furanische Lipide und polyhydroxylierte Fettalkohole von Avocado umfasst, erhält, wobei die Temperatur der Molekulardestillation zwischen 100 und 160°C eingeschlossen liegt und der Molekulardestillations-Druck zwischen 10⁻³ und 5.10⁻² mmHg eingeschlossen liegt.

2. Verfahren nach Anspruch 1, **dadurch gekennzeichnet, dass** für den Schritt der Molekulardestillation, welcher jenem der Herstellung des unverseifbaren Anteils folgt, die Temperaturmittel für eine Temperatur zwischen 100 und 140°C eingeschlossen eingestellt werden und die Druckmittel für einen Druck zwischen 10⁻³ und 5.10⁻² mmHg eingeschlossen eingestellt werden, um ein Destillat, welches hauptsächlich furanische Lipide von Avocado umfasst, zu erhalten.

3. Verfahren nach Anspruch 1, **dadurch gekennzeichnet, dass** für den Schritt der Molekulardestillation, welcher jenem der Herstellung des unverseifbaren Anteils folgt, die Temperaturmittel für eine Temperatur zwischen 130 und 160°C eingeschlossen eingestellt werden und die Druckmittel für einen Druck zwischen 10⁻³ und 5.10-² mmHg eingeschlossen eingestellt werden, um ein Destillat, welches hauptsächlich furanische Lipide von Avocado und polyhydroxylierte Fettalkohole von Avocado umfasst, zu erhalten.

4. Verfahren nach einem der vorangegangenen Ansprüche, **dadurch gekennzeichnet, dass** der unverseifbare Anteil von Avocado ausgehend von der Frucht, welche vorab thermisch behandelt worden ist, hergestellt wird.

5. Verfahren nach Anspruch 4, **dadurch gekennzeichnet, dass** die Frucht vorab thermisch behandelt wird durch kontrollierte Trocknung bei einer Temperatur von wenigstens ungefähr 80°C während einer Zeitspanne von wenigstens ungefähr vier Stunden.

6. Verfahren nach einem der vorangegangenen Ansprüche, **dadurch gekennzeichnet, dass** der unverseifbare Anteil von Avocado ausgehend von dem Öl der Frucht, welches vorab durch Molekulardestillation an unverseifbarem Anteil angereicht worden ist, hergestellt wird.

7. Verfahren nach Anspruch 6, **dadurch gekennzeichnet, dass** die Molekulardestillation des Öls der Frucht bei einer Temperatur zwischen ungefähr 180 und ungefähr 230°C eingeschlossen und bei einem Druck zwischen ungefähr 10⁻³ und ungefähr 10⁻² mmHg eingeschlossen ausgeführt wird.

8. Verfahren nach einem der vorangegangenen Ansprüche, **dadurch gekennzeichnet, dass** die Molekulardestillation ausgeführt wird, indem eine Vorrichtung, die unter den Molekulardestillierapparaten vom Zentrifugen-Typ und den Molekularapparaten vom Typ derer, die unter Abschabung eines Films arbeiten, ausgewählt wird, eingesetzt wird.
